(19) Europäisches Patentamt
European Patent Office
Office européen des brevets

(11) Numéro de publication : **0 388 275 B1**

# (12) FASCICULE DE BREVET EUROPEEN

(45) Date de publication du fascicule du brevet : **03.02.93 Bulletin 93/05**

(51) Int. Cl.$^5$ : **A61K 33/30,** A61K 31/315

(21) Numéro de dépôt : **90400652.5**

(22) Date de dépôt : **13.03.90**

## (54) Compositions dermatologiques et cosmétologiques à base d'alcoylcarboxamide et de sel de zinc utiles dans le traitement de la dermite séborrhéique et/ou des troubles de la séborrhée.

(30) Priorité : **13.03.89 FR 8903235**

(43) Date de publication de la demande : **19.09.90 Bulletin 90/38**

(45) Mention de la délivrance du brevet : **03.02.93 Bulletin 93/05**

(84) Etats contractants désignés : **AT BE CH DE DK ES FR GB GR IT LI LU NL SE**

(56) Documents cités :
**EP-A- 0 250 300**
**EP-A- 0 281 812**
**EP-A- 0 288 342**
**DE-A- 2 517 413**
**US-A- 4 016 287**

(73) Titulaire : **PIERRE FABRE COSMETIQUE**
**45, Place Abel Gance**
**F-92100 Boulogne (FR)**

(72) Inventeur : **Fabre, Pierre**
**1, avenue d'Albi**
**F-81100 Castres (FR)**
Inventeur : **Jeanjean, Michel**
**14 Chemin Savignol**
**F-31220 Castanet Tolosan (FR)**
Inventeur : **Mouzin, Gilbert**
**11, rue des Pénitents Blancs**
**F-31000 Toulouse (FR)**

(74) Mandataire : **Ahner, Francis et al**
**CABINET REGIMBEAU, 26, avenue Kléber**
**F-75116 Paris (FR)**

**EP 0 388 275 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention, réalisée au Centre de Recherche Dermatologique et Cosmétologique Pierre FABRE, concerne de nouvelles formulations topiques utilisables en dermatologie et cosmetologie.

Ces formulations, à base d'une association alcoylcarboxamides et de sels de zinc, potentialisent les effets antiinflammatoires, antiprurigineux et antiséborrhéiques des principes actifs.

Des travaux récents ont montré que le zinc est impliqué dans de nombreux systèmes enzymatiques (PASSAD A.S. Clinical, endocrinological and biochemical effects of zinc deficiency - Clinics in endocrinology and metabolism. 14 567 - 589 - 1985) et son rôle dans le métabolisme de la testostérone a été également rapporté (LEAKE A., CHISHOLUM G.D., HABIB F.K. The effects of zinc on the 5 $\alpha$ reduction of testosterone by the hyperplastic human prospate gland). Journal or steroid biochemistry 25 supplement 67S, abstract 182,1986).

L'association des principes actifs utilisés dans les formulations topiques selon la présente invention permet d'obtenir des résultats performants dans le traitement des troubles séborrhéiques ainsi que dans les cas des dermites séborrhéiques.

De façon préférentielle, l'alcoylcarboxamide répond à la formule générale (I) suivante

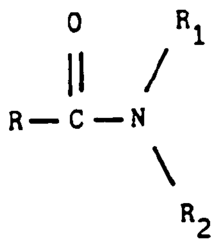

(I)

dans laquelle :
R représente un atome d'hydrogène, un groupe alcoyl en C1 à C5 ou un groupe pyridyle.

R1 et R2 peuvent être identiques ou différents et représentent un atome d'hydrogène ou un groupe alcoyl en C1 et C5 éventuellement substitué par un hydroxyle. Ces composés de formule I peuvent être préparés par des procédés chimiques connus de l'homme de l'art et sont mentionnés par la demanderesse dans le brevet 87 12788. Composition alopécique comportant du Minoxidil (Pierre FABRE, Henri COUSSE et Gilbert MOUZIN).

Dans le cadre de la présente invention, on préfère utiliser un alcoylcarboxamide répondant à la formule (II) suivante :

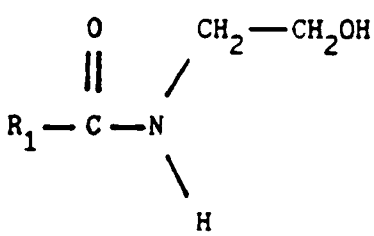

dans laquelle R1 est un groupe alcoyl en C1 à C5 et est de préférence le groupe méthyl et plus particulièrement :

le N (hydroxy 2 éthyl) acétamide

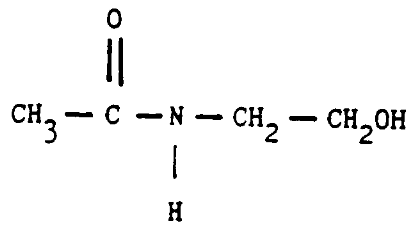

le N (hydroxy 3 propyl) acétamide

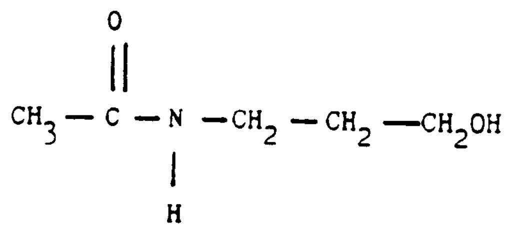

le N (hydroxy 2 éthyl) proprionamide

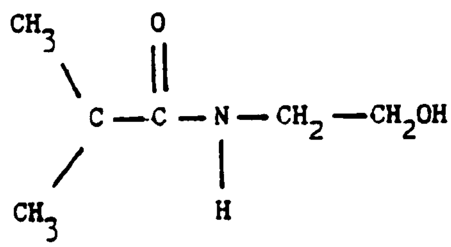

le N (hydroxy 2 éthyl) isopropylacétamide

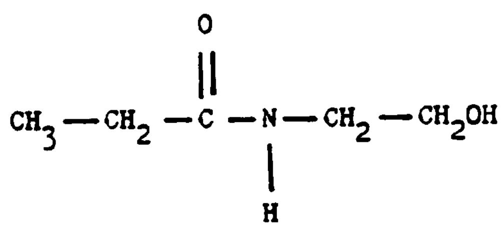

La composition selon la présente invention comporte en outre un sel de zinc d'acide minéral ou organique et plus particulièrement les dérivés suivants :

- sulfate de zinc
- chlorure de zinc
- acétate de zinc
- gluconate de zinc
- glycérophosphate de zinc
- glycinate de zinc
- lactate de zinc
- laurate de zinc
- oléate de zinc, et
- rétinoate de zinc (EP 038 246)

La composition selon l'invention contient avantageusement de 0,5 à 10% en poids d'alcoylcarboxamides et de 0,1 à 2% en poids de sels de zinc.

Les formulations topiques de la nouvelle association de principes actifs selon la présente invention sont illustrées par les exemples suivants :

EXEMPLE 1 Lotion capillaire

| | |
|---|---|
| Sulfate de zinc | 0,5 à 2% |
| N(hydroxy 2 éthyl)acétamide | 1 à 5% |
| Ethanol à 95% | 25% vol |
| Polymère de polyglycolpolyamine | 0,5 à 1% |
| Eau minéralisée | qsp 100 g |

EXEMPLE 2 Lotion visage

| | |
|---|---|
| Sulfate de zinc | 2% |
| N(hydroxy 2 éthyl)acétamide | 5% |
| Alcool éthylique | 11% vol |
| Eau d'Hamamelis | 20% |
| Polyéthylène glycol 300 | 3% |
| Imidazolidinyl urée | 0,15% |
| Eau déminéralisée | qsp 100 g |

EXEMPLE 3 Lotion visage

| | |
|---|---|
| Sulfate de zinc | 2% |
| Alcool éthylique | 11% vol |
| Eau d'Hamamelis | 20% |
| Polyéthylène glycol 300 | 3% |
| Imidazolidinyl urée | 0,15% |
| Eau déminéralisée | qsp 100g |

EXEMPLE 4 Emulsion souple

| | |
|---|---|
| Acétate de zinc | 0,5 à 2% |
| N(hydroxy 3 propyl) acétamide | 5 à 10% |
| Alcool cétostéarylique | 5 à 10% |
| Acide stéarique | 1 à 3% |
| Monopalmitate de glycol | 1 à 2% |
| Lanoline | 0,5 à 1% |
| Huile de vaseline | 5 à 10% |
| Glycérol | 1 à 2% |
| Eau déminéralisée | qsp 100 g |

EXEMPLE 5 Emulsion riche

| | |
|---|---|
| Gluconate de zinc | 0,15 à 1,5% |
| N(hydroxy 2 étyl) proprionamide | 2 à 5% |
| Monostéarate de glycérol | 7 à 10% |
| Alcool acétylique | 7 à 10% |
| Huile d'amande douce | 5 à 10% |
| Huile de noisette | 2 à 5% |
| Solution aqueuse de sorbitol (à70%) | 2 à 5% |
| Eau déminéralisée | qsp 100 g |

EXEMPLE 6 Emulsion crème

| | |
|---|---|
| Sulfate de zinc | 0,5 à 2% |
| N(hydroxy 2 éthyl) acétamide | 1 à 5% |
| Monostéarate de glycérol | 10 à 20% |
| Myristate de triéthoxymyristyle | 1 à 2% |
| Diisopropyl dimérate | 1 à 2% |
| Eau déminéralisée | qsp 100 g |

EXEMPLE 7 Shampooing

| | |
|---|---|
| Lactate de zinc | 1 à 2% |
| N(hydroxy 2 éthyl) acétamide | 1 à 5% |
| Alkylamide diméthylbétaine (30%) | 2 à 6% |
| Alkyléthoxylsulfosuccinate disodique (30%) | 10 à 20% |
| Alkyl ester sulfate de sodium (28%) | 10 à 25% |
| Polysorbate 20 | 2 à 5% |
| Distéarate de PEG 6000 | 2 à 5% |
| Eau | qsp 100 g |

EXEMPLE 8 Gel moussant nettoyant visage

| | |
|---|---|
| Sulfate de zinc | 0,2 à 1% |
| N(hydroxy 2 éthyl) acétamide | 1 à 5% |
| Alkyléthoxysulfosuccinate de sodium (30%) | 10 à 20% |
| Cocoyliséthionate (70%) | 5 à 10% |
| Cocamido isopropyl betaine (40%) | 2 à 5% |
| Myristyl éther de suif | 2 à 5% |
| Stéarate de diéthylène glycol | 1 à 2% |
| Monopalmitate de glycol | 0,5 à 1% |
| Glycérol | 1 à 2% |
| Eau déminéralisée | qsp 100 g |

EXEMPLE 9 Pain de toilette sans savon

| | |
|---|---|
| Sulfate de zinc | 1 à 2% |
| N(hydroxy 2 éthyl) acétamide | 1 à 3% |
| Huile vaseline épaisse | 0,7 à 1,5% |
| Glycérol | 1 à 3% |
| T1 02 | 0,2 à 0,5% |
| Base sulfate et hémisulfosuccinate d'alkyle et d'alcool gras | qsp 100 g |

CLINIQUE

Les formulations selon les exemples 2 et 3 ont été testées dans le traitement topique de la dermite séborrhéique.

Cette étude en ouvert a porté sur 40 cas (26 femmes et 14 hommes, agés de 12 à 60 ans) de dermites séborrhéiques traitées par 2 applications quotidiennes de cette lotion pendant 4 semaines.

L'affection était essentiellment localisée au niveau du visage avec desquamation et prurit dans 39 cas, érythème dans les 40 cas.

Les résultats de cette étude sont mentionnés dans le tableau suivant.

| Améliorations | Formulations 2 | Formulations 3 |
|---|---|---|
| Prurit | ++++ | ++ |
| Desquamation | ++++ | ++ |
| Erythème | +++ | + |

+ : pas d'amélioration

++ : légère amélioration ≥ 35%

+++ : amélioration ≥ 65%

++++ : très forte amélioration ≥ 80%

Ces résultats confirment la potentialisation très nette de l'activité du sulfate de zinc en présence de N(hydroxy 2 éthyl) acétamide.

## Revendications

### Revendications pour les Etats contractants suivants : AT, BE, CH, DE, DK, FR, GB, IT, LI, LU, NL, SE

1. Composition dermocosmétologique utile dans le traitement de la dermite séborrhéique et/ou de la séborrhée, caractérisée en ce qu'elle comporte à titre de principes actifs une association d'alcoylcarboxamide et de sel de zinc d'acide minéral ou organique et en ce que l'alcoylcarboxamide répond à la formule générale suivante :

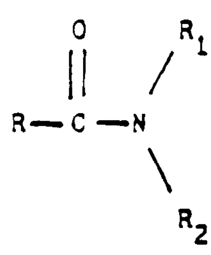

dans laquelle :

R représente un atome d'hydrogène, un groupe alcoyl en C1 à C5 ou un groupe pyridyle.

R1 et R2 peuvent être identiques ou différents et représentent un atome d'hydrogène ou un groupe alcoyl en C1 à C5 éventuellement substitué par un hydroxyle.

2. Composition selon la revendication 1, caractérisée en ce que l'alcoylcarboxamide répond à la formula sui-

vante :

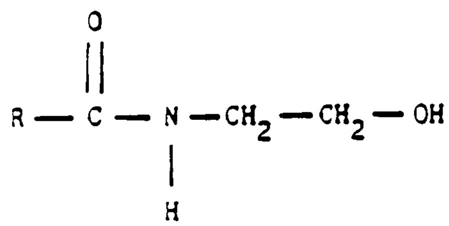

dans laquelle R est un groupe alcoyl en C1 à C5.

3. Composition selon laquelle la revendication 2, caractérisée en ce que l'alcoylcarboxamide est le N (hydroxy 2 éthyl) acétamide.

4. Composition selon la revendication 1, caractérisée en ce que de sel de zinc d'acide minéral est plus particulièrement le sulfate de zinc et/ou le chlorure de zinc.

5. Composition selon la revendication 1 caractérisée en ce que le sel de zinc d'acide organique est plus particulièrement :
   l'acétate de zinc
   le gluconate de zinc
   le glycérophosphate de zinc
   le glycinate de zinc
   le lactate de zinc
   le laurate de zinc
   l'oléate de zinc ou
   le rétinoate de zinc.

6. Composition selon l'une des revendications 1 à 5, caractérisée en ce qu'elle contient de 0,1 à 2% de sel de zinc.

7. Composition selon l'une des revendications 1 à 5, caractérisée en ce qu'elle contient de 0,5 à 10% d'alcoylcarboxamide.

**Revendications pour les Etats contractants suivants : ES, GR**

1. Procédé de préparation d'une composition dermocosmétologique utile dans le traitement de la dermite séborrhéique et/ou de la séborrhée, caractérisé en ce qu'on mélange, à titre de principes actifs une association d'alcoylcarboxamide et de sel de zinc d'acide minéral ou organique et en ce que l'alcoylcarboxamide répond à la formule générale suivante :

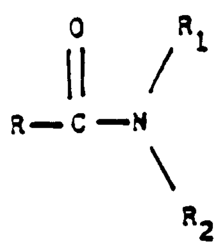

dans laquelle :
   R représente un atome d'hydrogène, un groupe alcoyl en C1 à C5 ou un groupe pyridyle.
   R1 et R2 peuvent être identiques ou différents et représentent un atome d'hydrogène ou un groupe alcoyl en C1 à C5 éventuellement substitué par un hydroxyle.

2. Procédé selon la revendication 1, caractérisé en ce que l'alcoylcarboxamide répond à la formule suivante :

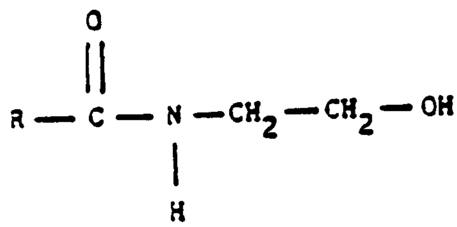

dans laquelle R est un groupe alcoyl en C1 à C5.

3. Procédé selon la revendication 2, caractérisé en ce que l'alcoylcarboxamide est le N (hydroxy 2 éthyl) acétamide.

4. Procédé selon la revendication 1, caractérisé en ce que le sel de zinc d'acide minéral est plus particulièrement le sulfate de zinc et/ou le chlorure de zinc.

5. Procédé selon la revendication 1, caractérisé en ce que le sel de zinc d'acide organique est plus particulièrement :
   l'acétate de zinc
   le gluconate de zinc
   le glycérophosphate de zinc
   le glycinate de zinc
   le lactate de zinc
   le laurate de zinc
   l'oléate de zinc ou
   le rétinoate de zinc.

6. Procédé selon l'une des revendications 1 à 5, caractérisé en ce que la composition contient de 0,1 à 2% de sel de zinc.

7. Procédé selon l'une des revendications 1 à 5, caractérisé en ce que la composition contient de 0,5 à 10% d'alcoylcarboxamide.

## Patentansprüche

**Patentansprüche für folgende Vertragsstaaten : AT, BE, CH, DE, DK, FR, GB, IT, LI, LU, NL, SE**

1. Hautkosmetische Zusammensetzung, die für die Behandlung der seborrhoischen Dermatitis und/oder der Seborrhoe verwendbar ist, dadurch gekennzeichnet, daß sie als aktive Prinzipien (Wirkstoffe) eine Kombination aus einem Alkylcarboxamid und einem Zinksalz einer Mineralsäure oder einer organischen Säure enthält und daß das Alkylcarboxamid die folgende allgemeine Formel hat:

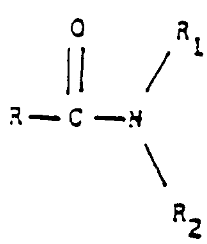

worin bedeuten:
   R ein Wasserstoffatom, eine $C_1$-$C_5$-Alkylgruppe oder eine Pyridylgruppe,
   $R_1$ und $R_2$, die gleich oder verschieden sein können, ein Wasserstoffatom oder eine $C_1$-$C_5$-Alkylgruppe, die gegebenenfalls durch ein Hydroxyl substituiert ist.

2. Zusammensetzung nach Anspruch 1, dadurch gekennzeichnet, daß das Alkylcarboxamid die folgende

Formel hat:

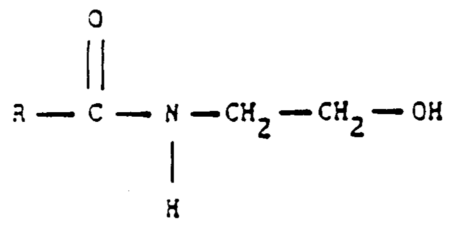

worin R eine $C_1$-$C_5$-Alkylgruppe darstellt.

3. Zusammensetzung nach Anspruch 2, dadurch gekennzeichnet, daß es sich bei dem Alkylcarboxamid um das N-(2-Hydroxy-ethyl)acetamid handelt.

4. Zusammensetzung nach Anspruch 1, dadurch gekennzeichnet, daß es sich bei dem Zinksalz einer Mineralsäure insbesondere um Zinksulfat und/oder Zinkchlorid handelt.

5. Zusammensetzung nach Anspruch 1, dadurch gekennzeichnet, daß es sich bei dem Zinksalz einer organischen Säure insbesondere handelt um
   Zinkacetat
   Zinkgluconat
   Zinkglycerophosphat
   Zinkglycinat
   Zinklactat
   Zinklaurat
   Zinkoleat oder
   Zinkretinoat.

6. Zusammensetzung nach einem der Ansprüche 1 bis 5, dadurch gekennzeichnet, daß sie 0,1 bis 2 % Zinksalz enthält.

7. Zusammensetzung nach einem der Ansprüche 1 bis 5, dadurch gekennzeichnet, daß sie 0,5 bis 10 % Alkylcarboxamid enthält.

**Patentansprüche für folgende Vertragsstaaten : ES, GR**

1. Verfahren zur Herstellung einer hautkosmetischen Zusammensetzung, die für die Behandlung der seborrhoischen Dermatitis und/oder der Seborrhoe geeignet ist, dadurch gekennzeichnet, daß man als aktive Prinzipien (Wirkstoffe) eine Kombination aus einem Alkylcarboxamid und einem Zinksalz einer Mineralsäure oder einer organischen Säure miteinander mischt, wobei das Alkylcarboxamid die folgende allgemeine Formel hat:

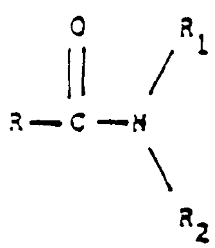

worin bedeuten:
   R ein Wasserstoffatom, eine $C_1$-$C_5$-Alkylgruppe oder eine Pyridylgruppe,
   $R_1$ und $R_2$, die gleich oder verschieden sein können, ein Wasserstoffatom oder eine $C_1$-$C_5$-Alkylgruppe, die gegebenenfalls durch ein Hydroxyl substituiert ist.

2. Verfahren nach Anspruch 1, dadurch gekennzeichnet, daß das Alkylcarboxamid die folgende Formel hat:

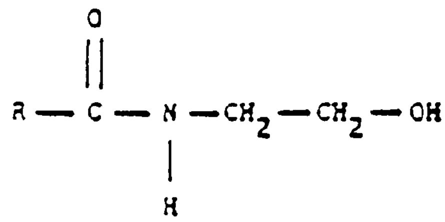

worin R eine $C_1$-$C_5$-Alkylgruppe darstellt.

3. Verfahren nach Anspruch 2, dadurch gekennzeichnet, daß es sich bei dem Alkylcarboxamid um das N-(2-Hydroxyethyl)acetamid handelt.

4. Verfahren nach Anspruch 1, dadurch gekennzeichnet, daß es sich bei dem Zinksalz einer Mineralsäure insbesondere um Zinksulfat und/oder Zinkchlorid handelt.

5. Verfahren nach Anspruch 1, dadurch gekennzeichnet, daß es sich bei dem Zinksalz einer organischen Säure insbesondere handelt um
   Zinkacetat
   Zinkgluconat
   Zinkglycerophosphat
   Zinkglycinat
   Zinklactat
   Zinklaurat
   Zinkoleat oder
   Zinkretinoat.

6. Verfahren nach einem der Ansprüche 1 bis 5, dadurch gekennzeichnet, daß die Zusammensetzung 0,1 bis 2 % Zinksalz enthält.

7. Verfahren nach einem der Ansprüche 1 bis 5, dadurch gekennzeichnet, daß die Zusammensetzung 0,5 bis 10 % Alkylcarboxamid enthält.

## Claims

### Claims for the following Contracting States : AT, BE, CH, DE, DK, FR, GB, IT, LI, LU, NL, SE

1. Dermocosmetological composition useful for the treatment of seborrhoeic dermatitis and/or seborrhoea, characterized in that it comprises as active ingredients a combination of an alkylcarboxamide and the zinc salt of a mineral or organic acid and in that the alkylcarboxamide corresponds to the following general formula :

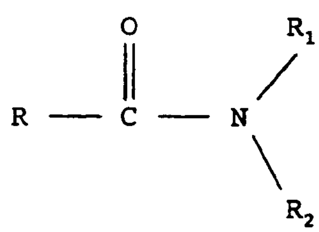

in which :

R represents a hydrogen atom, an alkyl group with C1 to C5 or a pyridyl group.

$R_1$ and $R_2$ may be identical to, or different from, each other and represent a hydrogen atom or an alkyl group with C1 to C5 possibly substituted with a hydroxyl.

2. Composition according to Claim 1, characterized in that the alkyl carboxamide corresponds to the following

formula :

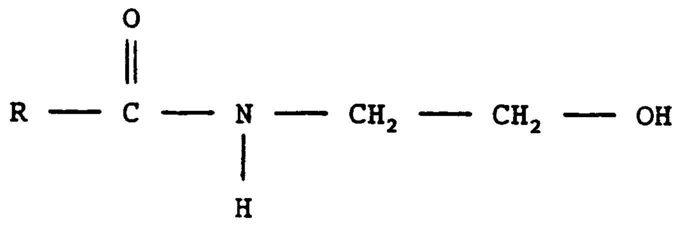

in which R is an alkyl group with C1 to C5.

3. Composition according to Claim 2, characterized in that the alkylcarboxamide is N (hydroxy 2 ethyl) acetamide.

4. Composition according to Claim 1, characterized in that the zinc salt of a mineral acid is more particularly zinc sulphate and/or zinc chloride.

5. Composition according to Claim 1, characterized in that the zinc salt of an organic acid is more particularly :
   Zinc acetate
   Zinc gluconate
   Zinc glycerophosphate
   Zinc glycinate
   Zinc lactate
   Zinc laurate
   Zinc oleate or
   Zinc retinoate.

6. Composition according to one of the Claims 1 to 5, characterized in that it contains from 0.1% to 2% zinc salt.

7. Composition according to one of the Claims 1 to 5, characterized in that it contains from 0.5% to 10% alkylcarboxamide.

**Claims for the following Contracting States : ES, GR**

1. Process for the preparation of a dermocosmetological composition useful for the treatment of seborrhoeic dermatitis and/or seborrhoea, characterized in that a mixture is prepared of active ingredients comprising a combination of an alkylcarboxamide and the zinc salt of a mineral or organic acid and in that the alkylcarboxamide corresponds to the following general formula :

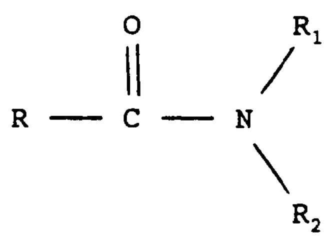

in which :
   R represents a hydrogen atom, an alkyl group with C1 to C5 or a pyridyl group.
   $R_1$ and $R_2$ may be identical to, or different from, each other and represent a hydrogen atom or an alkyl group with C1 to C5 possibly substituted with a hydroxyl.

2. Process according to Claim 1, characterized in that the alkyl carboxamide corresponds to the following formula :

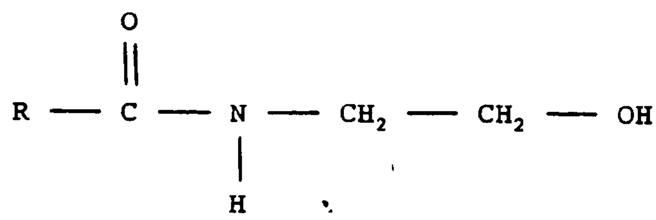

in which R is an alkyl group with C1 to C5.

3. Process according to Claim 2, characterized in that the alkylcarboxamide is N (hydroxy 2 ethyl) acetamide.

4. Process according to Claim 1, characterized in that the zinc salt of a mineral acid is more particularly zinc sulphate and/or zinc chloride.

5. Process according to Claim 1, characterized in that the zinc salt of an organic acid is more particularly :
   Zinc acetate
   Zinc gluconate
   Zinc glycerophosphate
   Zinc glycinate
   Zinc lactate
   Zinc laurate
   Zinc oleate or
   Zinc retinoate.

6. Process according to one of the Claims 1 to 5, characterized in that the composition contains from 0.1% to 2% zinc salt.

7. Process according to one of the Claims 1 to 5, characterized in that the composition contains from 0.5% to 10% alkylcarboxamide.